# EUROPEAN PATENT APPLICATION

(11) **EP 4 599 829 A1**
(43) Date of publication of application: **13.08.2025**
(21) Application number: 23874308.2
(22) Date of filing: 28.09.2023
(51) Int. Cl.: A61K 31/4164, A61P 37/02, A61P 17/00, A61P 9/00, A61P 11/00, A61P 13/08, A61P 13/10, A61P 1/16

(54) **USE OF IMIDAZOLIDINYL VANILLIC ACID ETHER DERIVATIVE IN TREATMENT OF DISEASE ACCOMPANIED BY FIBROSIS**

(30) Priority: 06.10.2022 CN 202211218683
(71) Applicant: Hefei Institute of Pharmaceutical Industry Co., Ltd., Hefai, Anhui 230088 (CN); Hefei Amvite Pharmaceutical Co., Ltd., Hefei, Anhui 230601 (CN)
(72) Inventor: CHU, Zhaoxing, Hefei, Anhui 230601 (CN); MO, Jiajia, Hefei, Anhui 230601 (CN); ZHAO, Yan, Hefei, Anhui 230601 (CN); LIN, Gaofeng, Hefei, Anhui 230601 (CN); SHAO, Li, Hefei, Anhui 230601 (CN); XU, Qinlong, Hefei, Anhui 230601 (CN); HE, Guangwei, Hefei, Anhui 230601 (CN)
(74) Representative: Hoffmann Eitle
(86) International application number: PCT/CN2023/122562
(87) International publication number: WO 2024/074116

(57) **Abstract**

The present invention relates to use of a compound represented by formula (I) or an ester thereof or a pharmaceutically acceptable salt thereof in the treatment of a disease accompanied by fibrosis.

## Description

### Technical Field

The present invention relates to the field of medicinal chemistry, and in particular relates to use of an imidazolidinyl vanillic acid ether derivative in the treatment of diseases accompanied by fibrosis.

### Background Art

Systemic fibrosis, especially systemic sclerosis (SSc), also known as "scleroderma", is a rare autoimmune disease with multiple organ involvement. Its pathological characteristics are fibrosis of the skin and internal organs accompanied by vascular lesions. Clinical manifestations include Raynaud's phenomenon, fingertip ulcers, pulmonary arterial hypertension or scleroderma renal crisis. The prevalence of systemic sclerosis worldwide is 8/1 million-56/1 million, with a higher incidence in females than in males (4: 1). There is still a lack of clear epidemiological data in China.

Although the etiology of systemic sclerosis is not fully understood, in recent years, great progress has been made in the study of abnormal immune activation, signal pathway conduction, cytokine release and the like involved in systemic sclerosis fibrosis. Chronic vascular damage, endothelial activation and immune activation are all considered to be the key to secondary fibroblast activation and related fibrosis (Nat Rev Rheumatol. 2019; 15(4): 208-24). Among them, vascular damage is an important part of the pathogenesis of systemic sclerosis, which can activate the coagulation cascade, activate platelets, increase the production of factors such as thrombin, thromboxane, platelet-derived growth factors, etc., and has a strong profibrotic effect.

Due to the rarity and clinical heterogeneity of systemic sclerosis, there is currently no specific therapeutic drug. Clinical treatment mainly focuses on improvement of organ function. The main measures include anti-inflammation and immunoregulation, circulation improvement and fibrosis inhibition. Therapeutic drugs are divided into immunomodulators and anti-fibrotic drugs. These drugs can alleviate disease progression but cannot improve the condition (Expert Opin Investig Drugs. 2021; 30(6): 635-652). New drugs for the treatment of systemic sclerosis still need to be developed clinically.

### Summary of the Invention

The present invention aims to provide the use of a compound represented by formula (I) 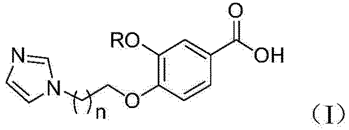 or an ester or a pharmaceutically acceptable salt thereof in the manufacture of a medicament for preventing and/or treating a disease accompanied by fibrosis,
wherein, R is selected from C₁ -C₆ alkyl group; n is an integer selected from 0, 1, 2, 3, 4, 5, and 6.

In certain embodiments of the present invention, the disease accompanied by fibrosis comprises systemic sclerosis.

In certain embodiments of the present invention, systemic sclerosis includes, but is not limited to, diffuse systemic sclerosis (e.g., diffuse cutaneous systemic sclerosis), localized systemic sclerosis (e.g., localized cutaneous systemic sclerosis), overlapping systemic sclerosis, and various lesions accompanied by systemic sclerosis.

In certain embodiments of the present invention, various lesions accompanied by systemic sclerosis include skin fibrosis, fibrosis of internal organs (such as kidneys, intestines, lungs, blood vessels, etc.), nephrogenic fibrotic skin lesions, nephrogenic systemic fibrosis, keloid formation, and systemic sclerosis related CRET syndrome (calcification, esophageal dysfunction, sclerosis and telangiectasia), vascular lesions, etc.

In certain embodiments of the present invention, vascular lesions accompanied by systemic sclerosis include, but are not limited to, vascular occlusive disease vasculitis, micro- and macrovascular lesions, Raynaud's phenomenon, ischemic digital lesions, digital ulcers, necrotic digital lesions, gangrene, and finger lesions.

In certain embodiments of the present invention, the disease accompanied by fibrosis also includes excess collagen (independent of the cause, such as autoimmune disease, chronic graft-versus-host disease, diabetes, poisoning, surgery, radiotherapy, etc.) leading to fibrosis of the skin, intestines, liver, lungs, heart, bladder, prostate, blood vessels, or other local or systemic fibrosis in tissues. Specifically, other fibrotic diseases include liver fibrosis caused by excess collagen, cirrhosis, pulmonary fibrosis, endomyocardial fibrosis, glomerulonephritis, interstitial renal fibrosis, fibrotic lesions caused by diabetes, myelofibrosis and similar fibrotic diseases, hypertrophic scars (including after surgery), etc.

### Beneficial Effects

The compounds of the present invention have good effects in treating diseases accompanied by fibrosis. Even when administered at low doses, the compounds of the present invention can achieve the desired therapeutic effects, indicating that the compounds of the present invention have good drug safety and have obvious clinical benefits when used as drugs.

### Brief Description of the Drawings

Figs. 1A and 1B show the effect of sodium 4-(2-(1H-imidazol-1-yl)ethoxy)-3-methoxybenzoate (HY-2) described in the present invention on skin thickening in model mice (mean ± SD, n = 8); ^{##} P < 0.01, vs blank group; * P < 0.05, ** P < 0.01 vs model group (bleomycin (BLM)).
Fig. 2 shows the effect of sodium 4-(2-(1H-imidazol-1-yl)ethoxy)-3-methoxybenzoate of the present invention on lung tissue pathology (HE staining) of model mice (mean ± SD, n=8).
Figs. 3A and 3B show the effect of sodium 4-(2-(1H-imidazol-1-yl)ethoxy)-3-methoxybenzoate described in the present invention on collagen deposition (Masson staining) in skin tissue of model mice (mean ± SD, n = 8); ^{##} P < 0.01, vs blank group; ** P < 0.01 vs BLM.
Figs. 4A and 4B show the effect of sodium 4-(2-(1H-imidazol-1-yl)ethoxy)-3-methoxybenzoate described in the present invention on collagen deposition (Masson staining) in lung tissue of model mice (mean ± SD, n = 8); ^{##} P < 0.01, vs blank group; ** P < 0.01 vs BLM.

### Detailed Description of the Invention

### Definition

As used in this specification, the following words and phrases are generally intended to have the meanings set forth below, unless the context in which they are used indicates otherwise.

As used herein, the term "alkyl" refers to a monovalent group having a straight or branched saturated hydrocarbon chain of 1 to 6 carbon atoms, more typically 1 to 5 carbon atoms, 1 to 4 carbon atoms, or 1 to 3 carbon atoms. This term is exemplified by groups such as methyl, ethyl, 1-propyl (n-propyl), 2-propyl (isopropyl), 1-butyl (n-butyl), 2-methyl-1-propyl (isobutyl), 2-butyl (sec-butyl), 2-methyl-2-propyl (tert-butyl), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, etc.

As used herein, the term "pharmaceutically acceptable salts" refers to salts that retain the biological effectiveness and properties of a given compound, and which are not biologically or otherwise undesirable. Pharmaceutically acceptable salts may be acid addition salts and/or base addition salts. Acid addition salts can be prepared from inorganic and organic acids. Salts derived from inorganic acids include hydrochlorides, hydrobromides, sulfates, nitrates, phosphates, carbonates, bisulfates, hydrophosphates, dihydrophosphates, bicarbonates, and the like; salts derived from organic acids include formates, acetates, propionates, glycolates, pyruvates, oxalates, malates, malonates, succinates, maleates, fumarates, tartrates, citrates, benzoates, cinnamates, mandelates, methanesulfonates, ethanesulfonates, p-toluenesulfonates, salicylates, lactates, nicotinates, lauryl sulfates, naphthalenesulfonates, camphorsulfonates, gluconates, glucuronates, oleates, palmitates, stearates, pamoates, trifluoroacetates, etc. Base addition salts can be formed with inorganic or organic bases. Salts derived from inorganic bases include sodium, potassium, ammonium, calcium, magnesium, iron, zinc, copper, lithium, barium, aluminum salts and the like; salts derived from organic bases include salts formed with various primary, secondary and tertiary amines, such as ethylamine, diethylamine, n-propylamine, isopropylamine, diethanolamine, meglumine, amino acids (e.g., lysine, arginine, glycine, etc.), piperazine, piperidine, morpholine, tromethamine, choline and the like.

As used herein, the term "pharmaceutically acceptable" means that the substance or composition must be chemically and/or toxicologically compatible with the other ingredients of those comprising the formulation and/or the mammal to be treated therewith.

As used herein, the term "therapeutically effective amount" refers to an amount sufficient to affect treatment as defined below when administered to a mammal in need of such treatment. The therapeutically effective amount will vary with the subject and disease condition being treated, the weight and age of the subject, the severity of the disease condition, the manner of administration, etc., and can be readily determined by one of ordinary skill in the art.

As used herein, the term "subject" is typically a human subject, although it is understood that the methods and/or uses described herein are also effective for other animals, such as mammals and vertebrate species. More specifically, the term "subject" can be applied to animals including, but not limited to, mice, rats, monkeys, dogs, pigs, and rabbits; as well as domestic swine (pigs and hogs), ruminants, horses, poultry, felines, bovines, rodents, canines, and the like.

As used herein, the term "treating" or "treatment" includes preventing a disease from occurring in an animal that may be susceptible to the disease but does not yet experience or show symptoms of the disease (prophylactic treatment), inhibiting a disease (slowing or arresting its development), alleviating the symptoms or side effects of a disease (including palliative treatment), and relieving a disease (causing regression of a disease).

As used herein, the term "parenteral" includes subcutaneous injections, intravenous, intramuscular, intrasternal injection or infusion techniques.

### Active compounds

The compound of the present invention is a compound represented by formula (I) 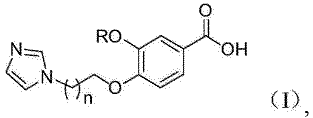 wherein, R is selected from C₁ -C₆ alkyl group; n is selected from integers of 0, 1, 2, 3, 4, 5, 6.

In certain embodiments of the present invention, the esters of the compound represented by formula (I) include C₁-C₆ alkyl esters, and the pharmaceutically acceptable salts of the compound represented by formula (I) include acid addition salts and base addition salts.

In certain embodiments of the present invention, the acid addition salts of the compound represented by formula (I) include inorganic acid salts and organic acid salts. In a preferred embodiment of the present invention, inorganic acid salts include but are not limited to hydrochloride, hydrobromide, sulfate, nitrate, phosphate, carbonate, bisulfate, hydrophosphate, dihydrophosphate, and bicarbonate; organic acid salts include but are not limited to formate, acetate, propionate, glycolate, pyruvate, oxalate, malate, malonate, succinate, maleate, fumarate, tartrate, citrate, benzoate, cinnamate, mandelate, methanesulfonate, ethanesulfonate, p-toluenesulfonate, salicylate, lactate, nicotinate, lauryl sulfate, naphthalenesulfonate, camphorsulfonate, gluconate, glucuronate, oleate, palmitate, stearate, pamoate, and trifluoroacetate.

In certain embodiments of the present invention, the base addition salts of the compound represented by formula (I) include salts formed with inorganic bases and salts formed with organic bases. In a preferred embodiment of the present invention, salts formed with inorganic bases include but are not limited to sodium salts, potassium salts, ammonium salts, calcium salts, magnesium salts, iron salts, zinc salts, copper salts, lithium salts, barium salts, and aluminum salts; salts formed with organic bases include but are not limited to salts formed with various primary amines, secondary amines, and tertiary amines, preferably include but are not limited to salts formed with ethylamine, diethylamine, n-propylamine, isopropylamine, diethanolamine, meglumine, amino acids (e.g., lysine, arginine, glycine, etc.), piperazine, piperidine, morpholine, tromethamine, and choline.

In certain embodiments of the present invention, R is selected from C₁ -C₃ alkyl group; preferably, R is selected from methyl, ethyl.

In certain embodiments of the present invention, n is selected from 0, 1 or 2; preferably, n is selected from 1.

In certain embodiments of the present invention, the compound represented by formula (I) is selected from sodium 4-(2-(1H-imidazol-1-yl)ethoxy)-3-methoxybenzoate, lithium 4-(2-(1H-imidazol-1-yl)ethoxy)-3-methoxybenzoate, calcium 4-(2-(1H-imidazol-1-yl)ethoxy)-3-methoxybenzoate, megluminium 4-(2-(1H-imidazol-1-yl)ethoxy)-3-methoxybenzoate, argininium 4-(2-(1H-imidazol-1-yl)ethoxy)-3-methoxybenzoate, hydrochloride of 4-(2-(1H-imidazol-1-yl)ethoxy)-3-methoxybenzoic acid, and fumarate of 4-(2-(1H-imidazol-1-yl)ethoxy)-3-methoxybenzoic acid.

### Pharmaceutical composition

The imidazolidinyl vanillic acid ether derivatives of the present invention can be administered via any pharmaceutically effective route. For example, it can be formulated into corresponding preparations for oral, intranasal, rectal, vaginal, sublingual, buccal, parenteral or transdermal administration.

In certain embodiments, the imidazolidinyl vanillic acid ether derivatives of the present invention can be formulated into pharmaceutically acceptable oral dosage forms, including but not limited to oral solid dosage forms and oral liquid dosage forms. Oral solid dosage forms may include, but are not limited to, tablets, capsule bases, caplets, powders, pulveres, pills, granules, and any combination thereof. These oral solid dosage forms can be formulated as immediate-release preparations, controlled-release preparations, sustained (extended)-release preparations, or modified-release preparations as desired. The oral solid dosage form of the present invention may also contain pharmaceutical excipients, such as fillers, diluents, lubricants, surfactants, glidants, binders, dispersants, suspending agents, disintegrants, viscosity enhancers, film formers, granulation aids, flavoring agents, sweeteners, coating agents, solubilizers, and combinations thereof. Depending on the desired release characteristics, the oral solid dosage form of the present invention may contain appropriate amounts of controlled-release agents, extended-release agents, modified-release agents, and the like. Oral liquid dosage forms include, but are not limited to, solutions, emulsions, suspensions, syrups, and any combination thereof. These oral liquid dosage forms can be formulated with any pharmaceutical excipient known to those skilled in the art for preparing liquid dosage forms, such as water, glycerin, syrup, alcohol, and any combination thereof.

**In** certain embodiments of the present invention, the imidazolidinyl vanillic acid ether derivatives of the present invention can be formulated into dosage forms suitable for parenteral use, including but not limited to lyophilized powders, solutions, suspensions (e.g., depot suspensions). **In** other embodiments, the compounds of the present invention may be formulated into topical dosage forms including, but not limited to, patches, gels, pastes, creams, emulsions, liniments, balms, lotions, ointments, and the like.

### Drug Administration

In certain embodiments of the present invention, provided is a method for preventing and/or treating a disease accompanied by fibrosis by administering a therapeutically effective amount of an imidazolidinyl vanillic acid ether derivative of the present invention to a subject in need thereof.

Administration of the imidazolidinyl vanillic acid ether derivatives of the present invention can be achieved by any therapeutically useful route of administration, including but not limited to oral, intranasal, rectal, vaginal, sublingual, buccal, parenteral or transdermal administration. The dosage administered should be adjusted according to the age, weight and condition of the subject, as well as the route of administration, dosage form and regimen, and the desired results. **In** order to obtain the desired plasma concentration of the compound of the present invention for the treatment or prevention of diseases accompanied by fibrosis, the daily dose of the compound of the present invention is preferably in the range of about 0.1 mg/day to about 5000 mg/day. In certain embodiments, the dosage of the compounds of the present invention is preferably in the range of about 0.5 mg/day to about 2000 mg/day; about 1 mg/day to about 1000 mg/day; about 1 mg/day to about 500 mg/day; about 1 mg/day to about 100 mg/day; about 1 mg/day to about 50 mg/day; or about 1 mg/day to about 20 mg/day.

### Drug Combination

The imidazolidinyl vanillic acid ether derivatives of the present invention can be administered in combination with one or more additional therapeutic agents for the prevention and/or treatment of diseases accompanied by fibrosis. In certain embodiments of the present invention, the additional therapeutic agent may be an adrenal corticosteroid, an anti-fibrotic agent, an immunosuppressant, a proton pump inhibitor, an angiotensin converting enzyme inhibitor, an endothelin receptor antagonist, a prostacyclin derivative, a cannabinoid receptor type 2 antagonist, or an IL-17 pathway inhibitor.

In certain embodiments of the present invention, the adrenal corticosteroid includes, for example, prednisolone.

In certain embodiments of the present invention, the anti-fibrotic agent includes, for example, pirfenidone and nintedanib.

In certain embodiments of the present invention, the immunosuppressant includes, for example, cyclophosphamide, mycophenolate mofetil, cyclosporine, tacrolimus, azathioprine, mizoribine, and methotrexate.

In certain embodiments of the present invention, the proton pump inhibitor includes, for example, omeprazole, lansoprazole, rabeprazole, and esomeprazole.

In certain embodiments of the present invention, the angiotensin converting enzyme inhibitor includes, for example, captopril, enalapril, alacepril, imidapril, and temocapril.

In certain embodiments of the present invention, the endothelin receptor antagonist includes, for example, bosentan, ambrisentan, and macitentan.

In certain embodiments of the present invention, the prostacyclin derivative includes, for example, iloprost, beraprost, treprostinil, epoprostenol, or clinprost.

In certain embodiments of the present invention, the cannabinoid receptor type 2 antagonist includes, for example, Lenabasum.

In certain embodiments of the present invention, the IL-17 pathway inhibitor is an anti-IL-17RA antibody, an anti-IL-17A antibody, an anti-IL-17A/F antibody, an anti-IL-23p40 subunit antibody, and/or an anti-IL-23p19 subunit antibody, such as berdalumab, secukinumab, ixekizumab, nitakinumab, bimegizumab, ustekinumab, tirazumab, risakizumab, migizone, brevitzumab, or guselkumab.

The compounds of the present invention and one or more additional therapeutic agents may be administered simultaneously or at different time; they may be administered together in the same formulation or separately in different formulations.

The present invention is further illustrated by the following specific examples, which are not intended to limit the present invention. The following specific examples use sodium 4-(2-(1H-imidazol-1-yl)ethoxy)-3-methoxybenzoate as a representative compound of the imidazolidinyl vanillic acid ether derivatives of the present invention to evaluate the biological activities of the compounds of the present invention.

### Examples

### Example 1 Synthesis of Compound sodium 4-(2-(1H-imidazol-1-yl)ethoxy)-3-methoxybenzoate (HY-2)

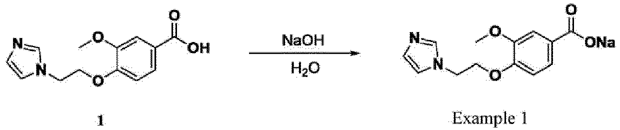

Compound 1 (200 mg, 0.76 mmol) and water (2 mL) were added to a 25 mL single-necked flask, and NaOH (31 mg, 0.76 mmol) was added under an ice-water bath, and the mixture was reacted at room temperature for 2 h. The reaction solution was slowly added dropwise to acetone (20 mL), and a white solid precipitated. After the addition, the mixture was stirred at room temperature for 1 h. The mixture was filtered, washed with acetone (2 mL x 3), and dried to obtain 190 mg of a white solid compound with a yield of 87.7%.

¹H NMR (500MHz, D₂O) δ (ppm): 7.70 (s, 1H, ArH), 7.47 (s, 1H, ArH), 7.44 (d, =9.0Hz, 1H, ArH), 7.19 (s, 1H, ArH), 6.97 (s, 1H, ArH), 6.92 (d, J = 8.5Hz, 1H, ArH), 4.39 (s, 4H, CH₂CH₂), 3.82 (s, 3H, OCH₃).

### Example 2 Synthesis of the compound lithium 4-(2-(1H-imidazol-1-yl)ethoxy)-3-methoxybenzoate

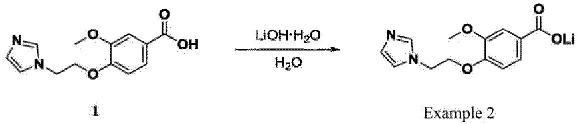

Compound 1 (200 mg, 0.76 mmol) and water (2 mL) were added to a 25 mL single-necked flask, and LiOH·H₂O (36 mg, 0.77 mmol) was added under an ice-water bath, and the mixture was reacted at room temperature for 2 h. The reaction solution was slowly added dropwise to acetone (20 mL), and a white solid precipitated. After the addition, the mixture was stirred at room temperature for 1 h. The mixture was filtered, washed with acetone (2 mL x 3), and dried to obtain 140mg of a white solid compound with a yield of 68.5%.

¹H NMR(400MHz,D₂O)δ(ppm):7.73(t,J=1.2Hz, 1H,ArH),7.54-745(m,2H, ArH),7.24(d,J=1.4Hz, 1H,ArH),7.04-6.96(m,2H,ArH),4.46(br,4H,CH₂CH₂),3.87(s,3H, OCH₃).

### Example 3 Synthesis of calcium 4-(2-(1H-imidazol-1-yl)ethoxy)-3-methoxybenzoate

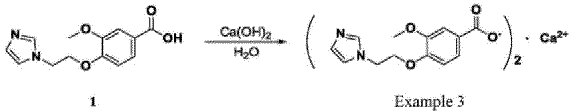

Compound 1 (100 mg, 0.38 mmol), water (2 mL) and calcium hydroxide (14 mg, 0.19 mmol) were added sequentially into a 10 mL single-necked flask, and the mixture was reacted at room temperature overnight under nitrogen protection. The reaction solution was slowly added dropwise to acetone (10 mL) at room temperature and stirred for 1 h. The mixture was filtered and dried to obtain 80 mg of a white solid compound with a yield of 37.3%.

¹H NMR(400MHz,D₂)O)δ(ppm):7.72(s,2H,ArH),7.49-7.46(m,4H,ArH), 7.21(s,2H,ArH),7.00(s,2H,ArH),6.92(d,J=8.4Hz,2H,ArH),4.41(s,8H,CH₂CH₂),3.84(s, 6H,OCH₃).

### Example 4 Synthesis of megluminium 4-(2-(1H-imidazol-1-yl)ethoxy)-3-methoxybenzoate

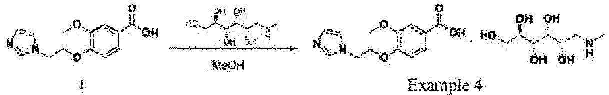

Compound 1 (100 mg, 0.38 mmol), meglumine (74 mg, 0.38 mmol) and methanol (2 mL) were added in sequence into a 25 mL single-necked flask, the mixture was reacted at 60° C for 1 h, and concentrated to obtain 160 mg of a white solid compound with a yield of 92.0%.

¹H NMR(400MHz,D₂O)δ(ppm):7.66(s,1H,ArH),7.42-7.36(m,2H,ArH),7.14(s,1H,ArH),6.92(s,1H,ArH),6.86(d,J=8.3Hz,1H,ArH),4.33(br,4 H,CH₂CH₂),4.02-3.98(m,1H,CH),3.75(s,3H,OCH₃),3.74-3.72(m,1H,CH),3.72-3.71(m,1H,CH),3.70-3.64(m,1H,CH),3.60-3.56(m,1H,H of CH₂),3.56-3.53(m,1H,H of CH₂),3.15-3.02(m,2H,CH₂),2.66(s,3H,NHCH₃).

### Example 5 Synthesis of argininium 4-(2-(1H-imidazol-1-yl)ethoxy)-3-methoxybenzoate

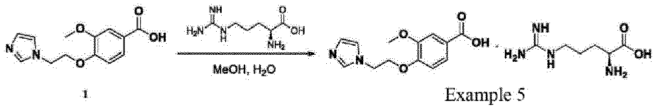

Compound 1 (100 mg, 0.38 mmol) and methanol (2 mL) were added to a 25 mL single-necked flask in sequence. After the addition was completed, the mixture was stirred until completely dissolved. Then, a solution of arginine (66 mg, 0.38 mmol) in water (2 mL) was added dropwise to the reaction solution. After the addition was completed, the mixture was stirred at 60°C for 1 h. The solvent was distilled off under reduced pressure to obtain a colorless oil, to which is added isopropyl ether (10 mL×3) and evaporated to obtain a white powder, which was dried in vacuo at 45° C for 5 h to obtain 150 mg of a white solid compound with a yield of 90.4%.

¹H NMR(400MHz,D₂O)δ(ppm):7.79(s,1H,ArH),7.37(d,J=8.2Hz,2H,ArH),7.17(s,1H,ArH ),6.96(s,1H,ArH),6.82(d,J=8.2Hz,1H,ArH),4.36-4.32(m,2H,NCH₂),4.31-4.27(m,2H,OCH₂),3.73(s,3H,OCH₃),3.65(t,J=6.1Hz,1H,H of CHNH₂),3.10(t,J=6.9Hz,2H,NHCH₂),1.85-1.74(m,2H,CH₂),1.68-1.47(m,2H,CH₂).

### Example 6 Synthesis of 4-(2-(1H-imidazol-1-yl)ethoxy)-3-methoxybenzoic acid hydrochloride

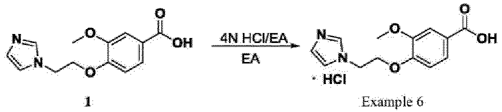

Compound 1 (200 mg, 0.76 mmol) and ethyl acetate (2 mL) were added to a 25 mL single-necked flask, and a solution of HCl in ethyl acetate (4N, 1 mL) was added dropwise under an ice-water bath, and the mixture was stirred at room temperature for 1 h. The reaction solution was filtered, washed with ethyl acetate (2 mL x 3), and dried to obtain 220 mg of a white solid compound with a yield of 96.7%.

¹H NMR(400MHz,D₂O)δ(ppm):8.83(t,J=14Hz,1H,ArH),7.64-7.46(m,4H,ArH),7.08-7.00(m,1H,ArH),4.70-4.68(m,2H,NCH₂),4.57-4.54(m,2H,OCH₂),3.81(br,3H,OCH₃).

HRMS (ESI): m/z [MH] - C₁₃H₁₅ClN₂O₄ Calculated: 297.0642; Found: 297.0632.

### Example 7 Synthesis of 4-(2-(1H-imidazol-1-yl)ethoxy)-3-methoxybenzoic acid fumarate

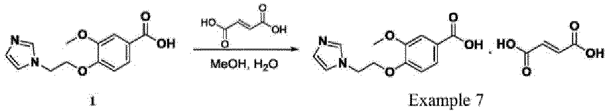

Compound 1 (100 mg, 0.38 mmol), fumaric acid (44 mg, 0.38 mmol), methanol (2 mL) and water (2 mL) were added in sequence to a 25 mL single-necked flask. After the addition was complete, the mixture was stirred at 60° C for 1 h. The solvent was distilled off under reduced pressure to obtain a white powder, which was then dried in vacuo at 45°C for 5 h to obtain 132 mg of a white solid compound with a yield of 92.1%.

H NMR (400MHz,D₂O) δ (ppm):8.66 (t,J=1.5Hz,1H,ArH), 7.45 (t,J=1.8Hz,1H,ArH), 7.39 (dd,J₁=8.5Hz,J₂=2.0Hz,1H,ArH), 7.33-7.29 (m,2H,ArH), 6.82 (d,J=8.5Hz,1H,ArH), 6.52 (s,2H,CH=CH), 4.54-4.50 (m,2H,NCH₂), 4.37-4.33 (m,2H,OCH₂), 3.67 (s,3H,OCH₃).

### Example 8: Effects of Imidazolidinyl Vanillic Acid Ether Derivatives on Skin Thickening in BLM-Induced Systemic Sclerosis Mouse Model

### 1.1 Materials and methods

Female BALB/c mice, weighing 18-22 g, were purchased from Beijing Weitong Lihua Experimental Animal Technology Co., Ltd. and housed in an SPF environment for 1 week (with free access to drinking water during the feeding period). They were then randomly divided into 5 groups, 8 mice in each group, namely, blank group, model group, 15 mg/kg, 30 mg/kg, and 60 mg/kg dosage groups of sodium 4-(2-(1H-imidazol-1-yl)ethoxy)-3-methoxybenzoate (HY-2). The back of each group of mice was shaved in an area of 1 cm*1 cm. Except for the blank group which was subcutaneously injected with normal saline, all other groups were injected with BLM 100 µL (0.5 mg/mL) for 28 consecutive days (the injection area was the four corners of the shaved area for the first 4 injections, and the center of the shaved area for the 5th injection, and this cycle was repeated. The skin changes at the subcutaneous injection site and the weight changes of the mice were observed every week). After the injection of BLM, the treatment group was orally (gavaged) given the corresponding dose of sodium 4-(2-(1H-imidazole-1-yl)ethoxy)-3-methoxybenzoate, once a day, for 28 consecutive days.

### 1.2 Experimental Results

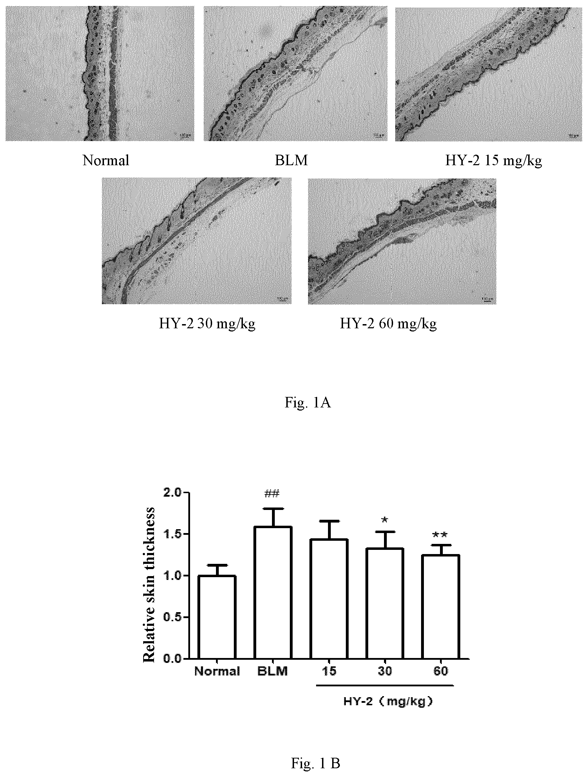

After the last administration, the animals were sacrificed, and appropriate skin tissues were taken for HE staining and measurement of skin thickening. The results showed that the skin of the mice in the model group was significantly thickened and subcutaneous fat was lost; compared with the model group, the skin thickening of the mice in the HY-2 30 and 60 mg/kg dose groups was significantly inhibited (P<0.05, P<0.01) *(see* Figs. 1A and 1B).

Example 9: Effect of Imidazolidinyl Vanillic Acid Ether Derivatives on Lung Histopathology in BLM-Induced Systemic Sclerosis Mouse Model

### 2.1 Materials and methods

Female BALB/c mice, weighing 18-22 g, were purchased from Beijing Weitonglihua Experimental Animal Technology Co., Ltd. and housed in an SPF environment for 1 week (with free access to water during the feeding period). They were then randomly divided into 5 groups, with 8 mice in each group, namely, blank group, model group, HY-2 15 mg/kg, 30 mg/kg, and 60 mg/kg dose groups. The back of each group of mice was shaved in an area of 1 cm*1 cm. Except for the blank group which was subcutaneously injected with normal saline, all other groups were injected with BLM 100 µL (0.5 mg/mL) for 28 consecutive days (the injection area was the four corners of the shaved area for the first 4 injections, and the center of the shaved area for the 5th injection, and this cycle was repeated. The skin changes at the subcutaneous injection site and the weight changes of the mice were observed every week). After the injection of BLM, each dose treatment group was orally (gavage) given the corresponding dose of HY-2 once a day for 28 consecutive days.

### 2.2 Experimental Results

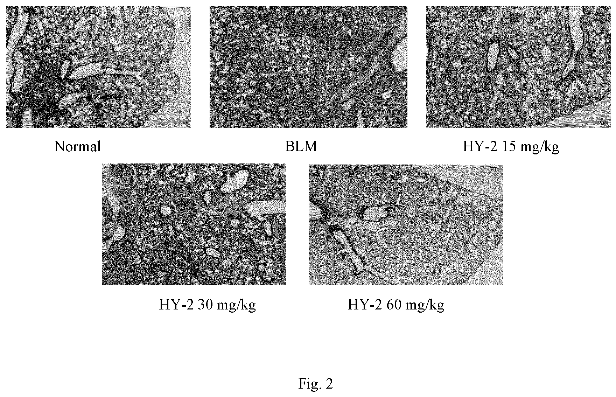

After the last administration, the animals were sacrificed, and appropriate lung tissues were taken for HE staining and slide preparation to examine the lung tissue structure and inflammatory cell infiltration. The results showed that the lung tissue structure of mice in the model group was disordered and infiltrated by inflammatory cells; compared with the model group, the lung tissue structure of mice in each dose group of HY-2 was regular and infiltrated by less inflammatory cells (*see* Fig. 2).

Example 10: Effect of Imidazolidinyl Vanillic Acid Ether Derivatives on Collagen Deposition in Skin and Lung Tissues of BLM-Induced Systemic Sclerosis Mouse Model

### 3.1 Materials and methods

Female BALB/c mice, weighing 18-22 g, were purchased from Beijing Weitonglihua Experimental Animal Technology Co., Ltd. and housed in an SPF environment for 1 week (with free access to water during the feeding period). They were then randomly divided into 5 groups, with 8 mice in each group, namely, blank group, model group, HY-2 15 mg/kg, 30 mg/kg, and 60 mg/kg dose groups. The back of each group of mice was shaved in an area of 1 cm*1 cm. Except for the blank group which was subcutaneously injected with normal saline, all other groups were injected with BLM 100 µL (0.5 mg/mL) for 28 consecutive days (the injection area was the four corners of the shaved area for the first 4 injections, and the center of the shaved area for the 5th injection, and this cycle was repeated. The skin changes at the subcutaneous injection site and the weight changes of the mice were observed every week). After the injection of BLM, each dose treatment group was orally (gavage) administered the corresponding dose of HY-2 once a day for 28 consecutive days.

### 3.2 Experimental Results

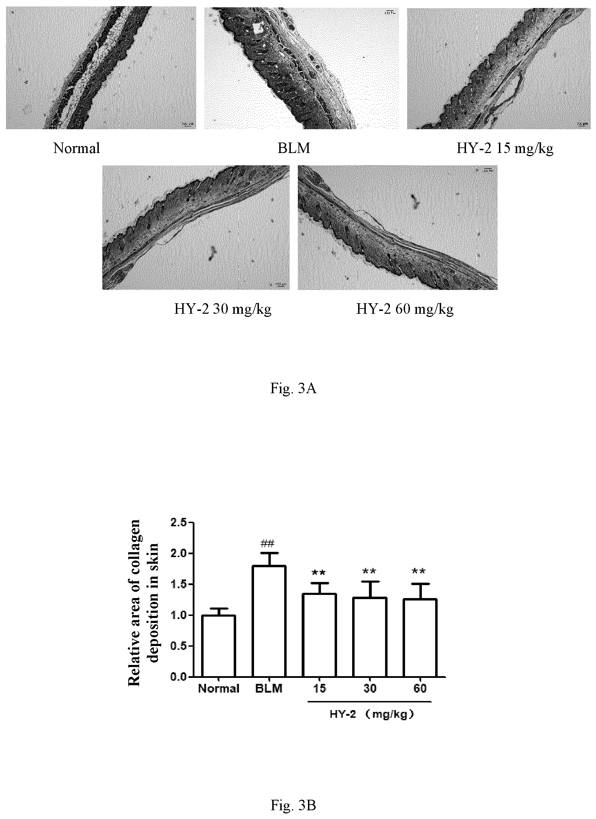

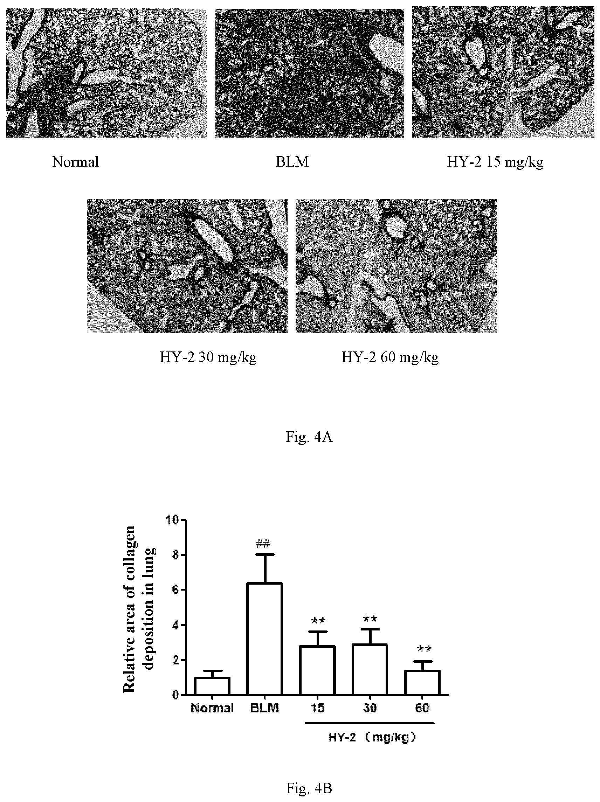

After the last administration, the animals were sacrificed, and appropriate skin and lung tissues were taken for Masson staining and slide preparation to examine the collagen deposition in the skin and lung tissues. The percentage of collagen deposition was quantified using Image-Pro^{®} Plus software. The results showed that obvious collagen deposition was found in the dermis of the skin and in the alveoli or blood vessels of the lung tissue of the mice in the model group (P<0.01). Compared with the model group, the collagen deposition in the skin and lung tissue of the mice in each dose group of HY-2 was significantly reduced (P<0.01) (*see* Figs. 3A, 3B, 4A, and 4B).

### Example 11: Comparison of the effect of imidazolidinyl vanillic acid ether derivatives and other antiplatelet drugs on skin thickening in the BLM-induced systemic sclerosis mouse model

### 1.1 Materials and methods

Female BALB/c mice, weighing 18-22 g, were purchased from Beijing Weitonglihua Experimental Animal Technology Co., Ltd. and housed in an SPF environment for 1 week (with free access to drinking water during the feeding period). They were then randomly divided into 9 groups, with 10 mice in each group, namely, blank group, model group, sodium ozagrel group, dipyridamole group, clopidogrel group, cilostazol group, ticagrelor group, aspirin group, and HY-2 group. The back of mice of each group was shaved in an area of 1 cm*1 cm. Except for the blank group which was subcutaneously injected with normal saline, all other groups were subcutaneously injected with BLM 100 µL (0.5 mg/mL) for 28 consecutive days (the injection area was injected at the four corners of the shaved area for the first 4 times, and the center of the shaved area for the 5th time, and this cycle was repeated. The skin changes at the subcutaneous injection site and the weight changes of the mice were observed every week). Each treatment group was respectively given sodium ozagrel, dipyridamole, clopidogrel, cilostazol, ticagrelor, aspirin, and HY-2 30 minutes after the injection of BLM, once a day for 28 consecutive days (the dosage and administration method are shown in Table 1).

**Table 1 Dosage and administration method**

| Experimental Group | Dosage (mmol/kg) | Administration method |
|---|---|---|
| Blank | \ | \ |
| Model Group | \ | \ |
| Sodium Ozagrel | 0.10 | Intravenous injection |
| Dipyridamole | 0.10 | Oral (gavage) |
| Clopidogrel | 0.10 | Oral (gavage) |
| Cilostazol | 0.10 | Oral (gavage) |
| Ticagrelor | 0.10 | Oral (gavage) |
| Aspirin | 0.10 | Oral (gavage) |
| HY-2 | 0.10 | Oral (gavage) |

### 1.2 Experimental steps and results

After the last administration, the animals were sacrificed, and the back skin tissue was taken, fixed with 4% paraformaldehyde, dehydrated, paraffin-embedded and sliced. The tissue sections were stained with HE, and the pathological morphological changes of the skin tissue were examined with a microscope and the skin thickening was measured. The experimental results are shown in Table 2. The results showed that

### Description

the skin thickness of mice in the BLM model group was significantly increased compared with that in the blank group (P<0.01), indicating that the model group was successfully established; compared with the model group, the skin thickness of mice in the HY-2 group was significantly decreased (P<0.01); compared with the sodium ozagrel group, dipyridamole group, clopidogrel group, cilostazol group, ticagrelor group and aspirin group, it had significant advantages in improving the increase in skin thickness of SSc mice induced by BLM (P<0.05, P<0.01). The experimental data were processed using GraphPad Prism 7, and the experimental groups were compared using the student t test. P < 0.05, and P < 0.01 indicated that the differences between the groups were statistically significant.

**Table 2 Effect of compounds on skin thickening in BLM-induced systemic sclerosis mouse model (mean ± SE, n = 8)**

| Experimental Group | Molar dose (mmol/kg) | Skin thickness (mm) |
|---|---|---|
| Blank | \ | 0.22±0.05 |
| Model Group | \ | 0.87±0.15^{#} |
| Sodium Ozagrel | 0.10 | 0.82±0.16^{aa} |
| Dipyridamole | 0.10 | 0.74+0.12^{a} |
| Clopidogrel | 0.10 | 0.81±0.10^{aa} |
| Cilostazol | 0.10 | 0.74±0.11^{a} |
| Ticagrelor | 0.10 | 0.74±0.07^{aa} |
| Aspirin | 0.10 | 0.80±0.09^{aa} |
| HY-2 | 0.10 | 0.59±0.06* |

| | | |
|---|---|---|
| ^{#}P<0.01 vs blank group; *P<0.01 vs model group; ^{a}P<0.05, ^{aa}P<0.01 vs HY-2 group. | | |

### Example 12: Comparison of the effect of imidazolidinyl vanillic acid ether derivatives and other antiplatelet drugs on the levels of thromboxane B2 (TXB2) and transforming growth factor β1 (TGF-β1) in the blood of BLM-induced systemic sclerosis mouse model

TGF-β1 is the strongest profibrotic mediator known to date. It was originally discovered in platelets, is usually upregulated after tissue injury, and plays a key role in the production of extracellular matrix. Platelets are an important source of TGF-β1. Platelet aggregation and activation activate TGF-β1 and release the same into blood vessels. At the same time, activated TGF-β1 can also enhance platelet aggregation. Activated TGF-β1 further accumulates near fibroblasts and initiates the fibrogenic process, leading to the final fibrosis of systemic sclerosis.

### 1.1 Materials and methods

Similar to Example 11. Mouse TXB2 ELISA kit was purchased from Shanghai Jianglai Biological, batch number: 20220715, antibody types: purified mouse TXB2 capture antibody and horseradish peroxidase (HRP)-labeled detection antibody; mouse TGF-β1 ELISA kit was purchased from Shanghai Jianglai Biological, batch number: 20220806, antibody types: purified mouse TGF-β1 capture antibody and horseradish peroxidase (HRP)-labeled detection antibody.

### 1.2 Experimental Principle

The kit uses a double-antibody one-step sandwich enzyme-linked immunosorbent assay (ELISA). Serum specimens, standards, and horseradish peroxidase (HRP)-labeled detection antibodies are added sequentially to the coated microwells pre-coated with mouse analyte capture antibodies, followed by incubation and thorough washing. The substrate TMB (3,3',5,5'-tetramethylbenzidine) is used for color development. TMB is converted to be blue under the catalysis of peroxidase (HRP) and then converted to be finally yellow under the action of acid. The depth of color is positively correlated with the content of the analyte in the serum sample. The absorbance (OD value) was measured at a wavelength of 450 nm using a microplate reader to calculate the sample concentration.

### 1.3 Experimental steps and results

After the last administration, the animals were sacrificed and serum samples were collected. Standard wells were set on the enzyme-labeled coated plate, standard solutions of different concentrations were prepared according to the instructions, and 50uL of standard solutions of different concentrations were added to each standard well. Blank wells and test sample wells were set on the enzyme-labeled coated plate, 40uL of sample diluent was first added to the test sample well, and 20uL of test serum sample was then added. 100uL of enzyme-labeled reagent was added to each well, except the blank well. The plate was sealed with a sealing film and incubated at 37°C for 60 minutes. The sealing film was removed, and after discarding the liquid and spinning dry, each well was filled with washing solution, and stood for 30 seconds, then discarding the washing solution, repeating 5 times and spinning dry. 50uL of color developer A (containing H₂O₂) was first added into each well, and then 50uL of color developer B (containing substrate TMB) was added, gently shaking to mix, and color developing at 37°C in the dark for 15 minutes. 50uL of stop solution was added to each well to stop the reaction (the blue color immediately turns to yellow). The microplate reader was adjusted to zero with the blank well, and the absorbance OD value of each well was measured in sequence at a wavelength of 450nm. The measurement should be carried out within 15 minutes after adding the stop solution. The experimental data were processed using GraphPad Prism 7. The experimental groups were compared using the student t test, with P < 0.05 and P < 0.01 indicating statistically significant differences between groups.

**Table 3 Effect of compounds on TXB2 and TGF-β1 in serum of BLM-induced systemic sclerosis mouse model (mean ± SE, n = 8)**

| Experimental Group | Molar dose (mmol/kg) | TXB2 (pg/mL) | TGF-β1 (pg/mL) |
|---|---|---|---|
| Blank | \ | 74.19±14.76 | 379.76±120.63 |
| Model Group | \ | 261.91±10.96^{#} | 913.10±64.73^{#} |
| Sodium Ozagrel | 0.10 | 196.54±45.30** | 884.74±33.45^{aa} |
| Dipyridamole | 0.10 | 248.84±38.88 | 867.56±113.73 |
| Clopidogrel | 0.10 | 208.46±42.87* | 822.62±76.60 |
| Cilostazol | 0.10 | 259.32±7.13^{a} | 838.27±65.93 |
| Ticagrelor | 0.10 | 197.76±42.08** | 847.62±43.73^{a} |
| Aspirin | 0.10 | 239.57±13.60* | 827.68±77.88 |
| HY-2 | 0.10 | 215.00±38.92* | 783.04±53.77** |

| | | | |
|---|---|---|---|
| ^{#} P<0.01 vs blank group; * P<0.05, ** P<0.01 vs model group; ^{a} P<0.05, ^{aa} P<0.01 vs HY-2 group. | | | |

The experimental results showed that TXB2 and TGF-β1 in the BLM model group were significantly higher than those in the blank control group (P<0.01), indicating that TXB2 and TGF-β1 play an important role in the BLM-induced systemic sclerosis model. The HY-2 group could significantly reduce the levels of TXB2 and TGF-β1 in serum (P<0.05, P<0.01). Although other antiplatelet drugs had certain inhibitory effect on TXB2 in serum, they had no significant inhibitory effect on the expression of TGF-β1. Among them, HY-2 had significant advantage over cilostazol in inhibiting serum TXB2 level (P<0.05); HY-2 was generally better than other drugs in inhibiting serum TGF-β1 level, especially had significant advantage compared with sodium ozagrel and ticagrelor (P<0.05, P<0.01).

In summary, the present invention uses the BLM-induced systemic sclerosis disease model to prove that HY-2 can effectively improve the symptoms of systemic sclerosis, inhibit pathological thickening of the skin, protect the structural integrity of lung tissue and reduce inflammatory cell infiltration, and has great potential for clinical application.

All references mentioned herein are incorporated by reference. It should be understood that many changes and modifications may be made to the technical solutions of the present invention without departing from the spirit and scope of the present disclosure.

## Claims

1. Use of compound represented by formula (I): 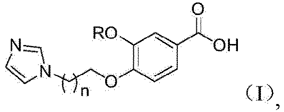
or an ester or a pharmaceutically acceptable salt thereof in the manufacture of a medicament for preventing and/or treating a disease accompanied by fibrosis, wherein
R is selected from C₁ - C₆ alkyl;
n is an integer selected from 0, 1, 2, 3, 4, 5, and 6.

2. The use according to claim 1, wherein the disease accompanied by fibrosis comprises systemic sclerosis.

3. The use according to claim 2, wherein the systemic sclerosis is selected from diffuse systemic sclerosis, localized systemic sclerosis, overlapping systemic sclerosis, and various lesions accompanied by systemic sclerosis.

4. The use according to claim 3, wherein the various lesions accompanied by systemic sclerosis comprise skin fibrosis, fibrosis of internal organs, nephrogenic fibrotic skin lesions, nephrogenic systemic fibrosis, keloid formation, systemic sclerosis related CRET syndrome, vascular occlusive disease vasculitis, micro- and macrovascular lesions, Raynaud's phenomenon, ischemic digital lesions, digital ulcers, necrotic digital lesions, gangrene, and finger lesions.

5. The use according to claim 2, wherein the disease accompanied by fibrosis comprises fibrosis of skin, intestine, liver, lungs, heart, bladder, prostate, blood vessels or other local or systemic fibrosis in tissues caused by excessive collagen.

6. The use according to claim 1, wherein the ester of the compound represented by formula (I) comprises C₁ - C₆ alkyl ester, and the pharmaceutically acceptable salt of the compound represented by formula (I) comprises acid addition salts and base addition salts.

7. The use according to claim 6, wherein the acid addition salts comprise inorganic acid salts and organic acid salts; wherein the inorganic acid salts comprise hydrochloride, hydrobromide, sulfate, nitrate, phosphate, carbonate, bisulfate, hydrophosphate, dihydrophosphate, and bicarbonate; and the organic acid salts comprise formate, acetate, propionate, glycolate, pyruvate, oxalate, malate, malonate, succinate, maleate, fumarate, tartrate, citrate, benzoate, cinnamate, mandelate, methanesulfonate, ethanesulfonate, p-toluenesulfonate, salicylate, lactate, nicotinate, lauryl sulfate, naphthalenesulfonate, camphorsulfonate, gluconate, glucuronate, oleate, palmitate, stearate, pamoate, trifluoroacetate; the base addition salts comprise salts formed with inorganic bases and salts formed with organic bases, wherein the salts formed with inorganic bases comprise sodium salt, potassium salt, ammonium salt, calcium salt, magnesium salt, iron salt, zinc salt, copper salt, lithium salt, barium salt, and aluminum salt, the salts formed with organic bases comprise salts formed with various primary amines, secondary amines, and tertiary amines, preferably comprising salts formed with ethylamine, diethylamine, n-propylamine, isopropylamine, diethanolamine, meglumine, amino acid, piperazine, piperidine, morpholine, tromethamine, and choline.

8. The use according to claim 1, wherein R is selected from methyl or ethyl.

9. The use according to claim 1, wherein n is an integer selected from 0, 1 or 2.

10. The use according to claim 1, wherein the compound represented by formula (I) is selected from sodium 4-(2-(1H-imidazol-1-yl)ethoxy)-3-methoxybenzoate, lithium 4-(2-(1H-imidazol-1-yl)ethoxy)-3-methoxybenzoate, calcium 4-(2-(1H-imidazol-1-yl)ethoxy)-3-methoxybenzoate, megluminium 4-(2-(1H-imidazol-1-yl)ethoxy)-3-methoxybenzoate, argininium 4-(2-(1H-imidazol-1-yl)ethoxy)-3-methoxybenzoate, hydrochloride of 4-(2-(1H-imidazol-1-yl)ethoxy)-3-methoxybenzoic acid, and fumarate of 4-(2-(1H-imidazol-1-yl)ethoxy)-3-methoxybenzoic acid.

## Amended claims

### Amended claims under Art. 19.1 PCT

1. [Amended] Use of compound represented by formula (I): 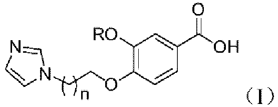
or a pharmaceutically acceptable salt thereof in the manufacture of a medicament for preventing and/or treating a disease accompanied by fibrosis, wherein
R is selected from C₁ - C₆ alkyl group;
n is an integer selected from 0, 1, 2, 3, 4, 5, and 6.

2. The use according to claim 1, wherein the disease accompanied by fibrosis comprises systemic sclerosis.

3. The use according to claim 2, wherein the systemic sclerosis is selected from diffuse systemic sclerosis, localized systemic sclerosis, overlapping systemic sclerosis, and various lesions accompanied by systemic sclerosis.

4. The use according to claim 3, wherein the various lesions accompanied by systemic sclerosis comprise skin fibrosis, fibrosis of internal organs, nephrogenic fibrotic skin lesions, nephrogenic systemic fibrosis, keloid formation, systemic sclerosis related CRET syndrome, vascular occlusive disease vasculitis, micro- and macrovascular lesions, Raynaud's phenomenon, ischemic digital lesions, digital ulcers, necrotic digital lesions, gangrene, and finger lesions.

5. The use according to claim 2, wherein the disease accompanied by fibrosis comprises fibrosis of skin, intestine, liver, lungs, heart, bladder, prostate, blood vessels or other local or systemic fibrosis in tissues caused by excessive collagen.

6. [Amended] The use according to claim 1, wherein the pharmaceutically acceptable salt of the compound represented by formula (I) comprises acid addition salts and base addition salts.

7. The use according to claim 6, wherein the acid addition salts compriseinorganic acid salts and organic acid salts; wherein the inorganic acid salts comprise hydrochloride, hydrobromide, sulfate, nitrate, phosphate, carbonate, bisulfate, hydrophosphate, dihydrophosphate, and bicarbonate; and the organic acid salts comprise formate, acetate, propionate, glycolate, pyruvate, oxalate, malate, malonate, succinate, maleate, fumarate, tartrate, citrate, benzoate, cinnamate, mandelate, methanesulfonate, ethanesulfonate, p-toluenesulfonate, salicylate, lactate, nicotinate, lauryl sulfate, naphthalenesulfonate, camphorsulfonate, gluconate, glucuronate, oleate, palmitate, stearate, pamoate, trifluoroacetate; the base addition salts comprise salts formed with inorganic bases and salts formed with organic bases, wherein the salts formed with inorganic bases comprise sodium salt, potassium salt, ammonium salt, calcium salt, magnesium salt, iron salt, zinc salt, copper salt, lithium salt, barium salt, and aluminum salt, the salts formed with organic bases comprise salts formed with various primary amines, secondary amines, and tertiary amines, preferably comprising salts formed with ethylamine, diethylamine, n-propylamine, isopropylamine, diethanolamine, meglumine, amino acid, piperazine, piperidine, morpholine, tromethamine, and choline.

8. The use according to claim 1, wherein R is selected from methyl or ethyl.

9. The use according to claim 1, wherein n is an integer selected from 0, 1 or 2.

10. The use according to claim 1, wherein the compound represented by formula (I) is selected from sodium 4-(2-(1H-imidazol-1-yl)ethoxy)-3-methoxybenzoate, lithium 4-(2-(1H-imidazol-1-yl)ethoxy)-3-methoxybenzoate, calcium 4-(2-(1H-imidazol-1-yl)ethoxy)-3-methoxybenzoate, megluminium 4-(2-(1H-imidazol-1-yl)ethoxy)-3-methoxybenzoate, argininium 4-(2-(1H-imidazol-1-yl)ethoxy)-3-methoxybenzoate, hydrochloride of 4-(2-(1H-imidazol-1-yl)ethoxy)-3-methoxybenzoic acid, and fumarate of 4-(2-(1H-imidazol-1-yl)ethoxy)-3-methoxybenzoic acid.
